# EUROPEAN PATENT APPLICATION

(11) **EP 3 181 066 A2**
(43) Date of publication of application: **21.06.2017**
(21) Application number: 16203860.8
(22) Date of filing: 31.01.2013
(51) Int. Cl.: A61B 17/15, A61B 17/17, A61F 2/46

(54) **TOOL**

(30) Priority: 09.02.2012 GB 201202298; 09.10.2012 GB 201218101
(62) Divisional of application: 13153565.0
(71) Applicant: MatOrtho Limited, Leatherhead, Surrey KT22 7BA (GB)
(72) Inventor: WATSON, Michael Andrew, Leatherhead, Surrey KT22 7BA (GB); TUKE, Michael Anthony, Leatherhead, Surrey KT22 7BA (GB)
(74) Representative: Gill, David Alan

(57) **Abstract**

The invention relates to a femoral jig (1) for use in knee surgery, said jig comprising a component (2) shaped to represent the overall profile of a femoral prosthetic component of a specific side and size, and having extending therefrom an anterior reference arm (3); said arm having a cutting guide (4) located thereon. It also relates to a tibial replacement thickness spacer (50) comprising a set of leaves and a tibial jig (20) for use in knee surgery, said jig comprising a component having tibial plate (21) and tibial posts (22) extending downwardly from said tibial plate and an anterior reference arm (23); said arm having a cutting guide (24) located thereon. Kits comprising these components are also described.

## Description

The present invention relates to tools for use in knee replacement operations. In one arrangement it relates to tools for use in total knee replacement operations. The invention also relates to a system or kit for use in knee replacement operations and to a method of carrying out such operations.

The knee joint is formed by the distal end of the femur, the proximal end of the tibia, a meniscus located therebetween and a patella. A plurality of ligaments, not only hold these components in the correct alignment, but also allow them to move relative to one another as the knee is flexed and extended. These ligaments also allow for some rotation. Since the knee supports several multiples of the entire weight of the body, and is subjected to various stresses as the body moves, it is vulnerable to damage through injury, disease or through the development of osteoarthritis. This damage causes loss of joint surface and hence pain.

When the knee has become damaged, the mobility of the body can be severely compromised. Various prostheses have therefore been suggested to replace the damaged natural joint. Whilst hinged components have been suggested it was realised that components which mimic the structure of the natural knee would be more appropriate.

Initial prostheses of this kind comprised a femoral component for location on the resected distal end of the femur and a tibial component for location on the proximal end of the resected tibia. Whilst these allowed for some articulation, the range of motion, and in particular the normal rotation and function of a healthy natural joint, was not achieved. With time, improved prostheses were suggested to address these problems.

For example, a so-called "total" knee prosthesis has been produced which comprises a tibial plate and a femoral component with an intervening meniscal bearing component which may be configured to have medial and lateral sides. Typically the tibial plate and femoral component are made from a suitable metal or metal alloy, such as an alloy of cobalt and chromium, whereas the meniscal bearing component is made from a synthetic plastics material, for example ultra high molecular weight polyethylene. In some designs the meniscal bearing component is fixed to the tibial plate. However, in other designs it is free to float at least to some extent with respect to the tibial plate in an attempt to mimic more closely the natural movement of the knee. In some arrangements the meniscal component may allow rotary and/or sliding motion on the tibial plate.

Whilst total knee implants are generally used, partial replacements are also known.

Generally, the equipment required to enable surgeons to perform these knee operations required to insert the prosthesis comprises several trays of metal and plastic components. These components include a variety of sizes of femoral and tibial components for the prosthesis and a large number of discrete and complex instruments which are required for the operation. Meniscal bearing components may also be present. Since the majority of the instruments are made of metal they are extremely heavy. This causes problems for theatre staff in moving them around. Further the trays in which they are provided, which are also made of metal so that they can be sterilized, have to be strong to support the weight of the components and thus they too are heavy which increases the overall weight needed to be handled with every case.

The trays also take up a large amount of space and are generally arranged and sterilized as a complete set that can be used in knee operations for any case of left or right knee from sizes small to large. As discussed above, the trays also contain trial components of the prosthesis. These components are provided by the manufacturing company in complete sets to cover all sizes and both knees. Thus any one set of trays can contain up to sixty components. The need for nursing staff to be able to quickly find the appropriate component for the particular need of the patient may be hampered by the number of components in the tray.

It will therefore be understood that a full set of the implant components and instruments must be provided for each operation to be carried out. These need to be cleaned and sterilized between each operation. Where hospitals carry out their sterilization on site, the time when the instruments are not available can be minimized but it is still significant. However, it is becoming increasingly common for sterilization to be carried out offsite which increases the time when the trays are not available for theatre use. The removal of the trays offsite may also lead to elements becoming misplaced or even mislaid. It will therefore be understood that the number of times each set of instruments and prosthesis can be used each week is decreased either meaning that additional sets have to be provided or that fewer operations can be carried out per week. However, since the cost of providing additional sets is often borne by the manufacture, this represents an economic burden for the manufacturer. The storage of additional bulky sets can be problematic for the hospital.

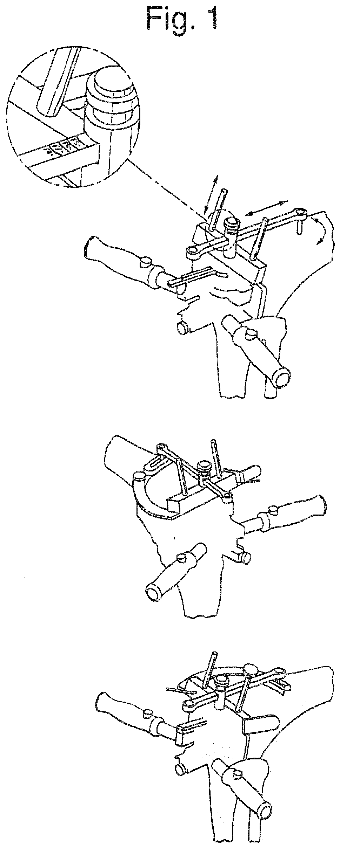

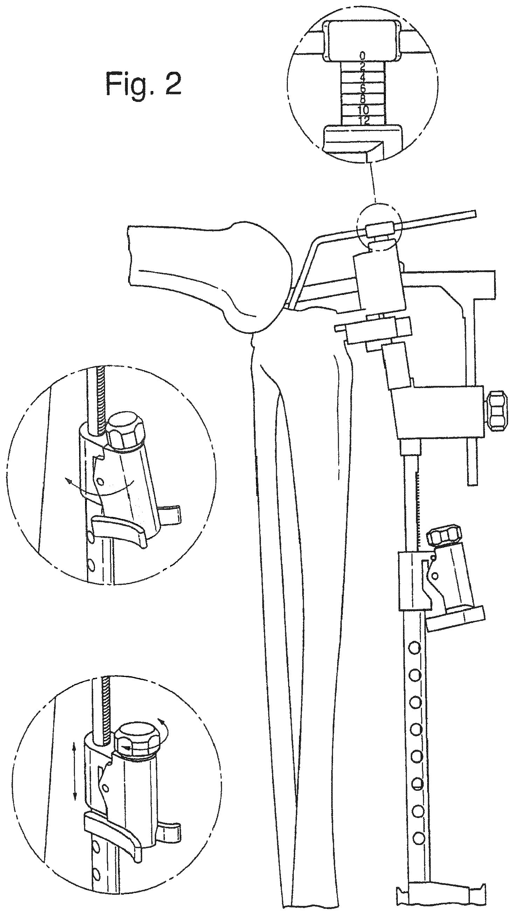

The systems currently available are quite complex and need the surgeon and nursing staff to be familiar with them. Examples of prior art femoral and tibial guides are illustrated in Figures 1 and 2 respectively. It can be seen that each guide includes a large number of adjustable components. This makes them difficult to use as the surgeon has to remember which adjuster carries out which function. The complexity of the guide and the ability to adjust a large number of different elements, can result in the guide being incorrectly positioned. Since the guide is used to guide the position of the cuts in the bone, incorrect positioning of the guide has serious consequences.

In addition to problems associated with the operation itself which may stem from unfamiliarity with the components and tools, the unfamiliarity may mean that the operation may take a lot longer than it should otherwise. The main disadvantage of this is to the patient but a further disadvantage relates to the longer use of expensive operating theatre time.

One problem which adds to the increase in time is the difficulty in finding which of 5 to 8 sizes of femoral component and a similar number of tibial components to use. Generally the selection of a size for the femoral component is carried out using jigs which take the position and size from the basic bony landmarks. This gives the nearest fit.

Since it is not possible to provide an infinite number of components there are inevitably steps between the sizes. Generally this is of the order of 3 to 4 mm. Unless a prosthesis is an exact fit, the surgeon will have to select the closest size. These step differences between sizes have to be accommodated in the bone and it is therefore necessary for a compromise to be made on size and fit.

Once an appropriately sized prosthesis is selected, it must be inserted with the correct alignment to provide correct functionality of the knee post-operatively.

Computer navigation systems have been suggested as a means to address some of the problems. It has been suggested that the use of such systems may lead to greater accuracy of leg alignment. However, these systems generally rely on a rod being inserted into the femoral canal to "find" its orientation. Whilst this process may go some way to addressing some of the alignment issues, insertion of the rod into the femur has various disadvantages and drawbacks. First the insertion of the rod into the femur provides a risk of damage to the femur. In addition the insertion of the rod into the femur risks pressurizing fat into the blood stream and opening a risky cavity for infection. A further problem with the rod arrangement is that unless it is aligned completely accurately and it is correctly related to hip position, it can lead to false alignments being taken.

An additional drawback with computer navigation systems is that they are expensive, cumbersome and take more time to use then the conventional systems. In addition, an equivalent level of accuracy of alignment can be found by aligning with the hip centre by simple manual means.

More recently it has been suggested that so-called "Patient Specific Instruments" (PSI) may offer an improved arrangement. In this system the patient is required to have MRI, CT scan, or both scans of the leg pre-operatively. In this connection it should be noted that whilst the cheaper X-ray can provide information as to the damage to the bone, little information relating to orientation can be gathered from an X-ray. It is therefore necessary in PSI systems for the expensive CT scans to be carried out.

The data from the scan is fed into computer software which also has details of the components for the manufacturer's implant. Whilst the next stage may be carried out by the surgeon, generally, a technician receives the patient's scan data and using the computer software chooses and aligns the correct components for the operation from the manufacturer's product. The technician is generally employed by the manufacturer and may be located anywhere in the world. The technician will generally not meet the surgeon or the patient.

The computer software enables a software plan to be developed. This is sent to the surgeon for review and sign-off. The manufacturer then uses the data which is now patient specific to create three-dimensional rapid prototype models of the patient's bones in the knee and guides for each bone. The guide model once made should fit and engage on the bone to provide lock on for the guide which is then pinned and provides a route to make the cuts on the two bones to provide most of the steps required in the operation.

The benefit of this system is that the manufacturer only has to supply the planned parts for use. This has the benefit of avoiding stock being tied up and also reduces the amount of space which is taken up in the operating theatre. In addition, operation time is reduced as selection of components from a set is not required. All of the PSI parts are disposed of post operation therefore obviating the requirement for post-operative sterilisation.

The main advantage of the PSI system to the hospital and patient is that fewer instruments are required for the operation and the time required for their use is potentially reduced and hence the whole operation takes less theatre time. Further a better accuracy of alignment is perceived and there is no requirement for a rod to be inserted into the femur.

The system also offers an advantage to the manufacturer in that a simpler instrument set can be provided. Further it locks the hospital and/or surgeon into using systems provided by the particular manufacturer. It also provides a new economic model for the company and the charging structure to the hospital.

Whilst PSI systems are finding favour and are being developed by a number of companies, there are various drawbacks and disadvantages. The first drawback is that the patient has to have an expensive MRI, CT or both scans which is not required for conventional systems.

A more serious problem is that a mistake somewhere in the data from the scan, or the analysis of the data by the technician could lead to problems which may not be noted until the operation is underway and at that time alternative equipment will not be available in the operating theatre or even in the hospital. A further problem may relate to the lock on of the guides which can lead to problems with alignment accuracy.

In any event, the surgeon still needs some of the old style instruments to carry out the operation and therefore the instruments have to be managed as part sets and as such many of the problems associated with conventional systems such as weight, sterilisation down time etc. apply equally here.

A serious issue is that one fundamental skill of the surgeon is being derogated to the technician. Whilst the surgeon will retain responsibility for the soft tissue work and responsibility for the patient, the surgery outcome relating to the implant is abrogated to the manufacturing company.

Whilst surgeon sign-off of the plan is required there is a risk of a longer term shift of the reality of the responsibility and ability to do the operation without the technical help.

A further problem is that the PSI system does not readily allow balancing of the knee during the operation. In an optimum system, the fit of the components should be assessed with the knee in both full extension and in flexion. If the knee is fitted so that the fit is optimized when extended then it may be too tight or unstable when in flexion which will mean the patient is unsteady when walking. The ability to assess and adjust the soft tissue is also removed. It is to assess this issue that knee balancing is carried out. Knee balancing can only be done during the operation and the effect of the balancing can be a change of the pre-operative plan which is difficult with the PSI system because it is essentially completed pre-operatively. There is a likelihood therefore that the balancing step will be omitted from the operation which may lead to reduced outcomes for the patient.

Whilst PSI planning may better optimize the sizing of a fixed femur implant increment by adjusting the component to fit 3-dimensionally to the bone, it is unlikely that manufacturers will utilize this fully.

Further the knee is a complex joint and there is a subtlety of knee proportions anterior to posterior and medial to lateral. The PSI system is unlikely to be able to address these subtle differences.

Even with PSI, once the bones have been cut it is still necessary to use a trial component to see whether the components are properly aligned and how thick any meniscal part of the tibial component needs to be. Thus the number of components required is still relatively high.

A still further problem with the PSI system is that at any particular time the operating theatre is prepared for a specific patient having a specific knee operated on. With conventional systems if the surgeon decides to do the other leg first he can. Similarly if a patient is unable to have the operation for any reason, the surgeon can simply move to the next patient. However, with PSI this cannot be done and due to the long lead time in having the PSI equipment produced it may be some time before another patient can be seen.

It is therefore desirable to provide instruments, systems and a method which address at least one or more of the above identified problems while providing the patient with a satisfactory outcome to the surgery.

In one arrangement a femoral guide is provided which allows the size of the selected femoral prostheses component to be tested in flexion and extension and between the two positions prior to making definitive bone cuts.

According to the first aspect of the present invention there is provided a femoral jig for use in knee surgery, said jig comprising a component shaped to represent the overall profile of a femoral prosthetic component of a specific side and size, and having extending therefrom an anterior reference arm; said arm having a cutting guide located thereon or means for attaching a cutting guide thereto.

The overall profile of the femoral prosthetic component will generally have distal and posterior surfaces and an interconnecting profile which mimics the prosthetic function with the tibia.

The jig will be configured as being either for a left knee or for a right knee.

Whilst the jig is described as comprising a component shaped and sized to represent the overall profile of the prosthetic component, it will be understood that it does not have to be the same shape provided that the main components of the joint such as the posterior and distal condyles and the anterior-proximal tip are represented.

The thickness of the femoral jig may be of any suitable size. In one arrangement the thickness posterior and distal is the same as the thinnest tibia component in an equivalent size less about 2mm on the medial side. Additionally or alternatively, the jig may have both posterior and distal thicknesses correct for 3 degrees external rotation. Thus it will be approximately 3mm thicker lateral than medial in flexion and extension. This means that sitting the component on unaffected cartilage distal and poster (medial or lateral) will provide correction of bone it replaces that has been cut from the tibia (as if that too included intact cartilage on a normal knee) in all four compartments i.e. medial, lateral, flex and extension with a 2 mm spacer added.

In general the component is shaped such that the posterior and distal surfaces that are against the femur are flat to ensure they find the two reference surfaces, however, the shape is generally rounded so that it will miss the femur at the half flexed position. In one arrangement, the external shape may be a conical cylinder.

The anterior proximal reference arm is shaped to sit at the ideal proximal lateral femoral component position on the femur ridge. Thus it is designed to be at the position to be taken by the femoral implant. In one arrangement, the reference arm may include adjustment means to allow the surgeon to visualize the fit of a prosthesis one size larger anteriorly. Any suitable adjustment means may be used. In one arrangement, the adjustment means may be a screw.

The jig optionally includes a handle extending upwardly from the jig. The handle may facilitate the surgeon adjusting the positioning of the jig. In one arrangement, a laser may be mounted on the handle or a mounting means for a separate laser may be provided. The laser may swivel in the femoral flexion extension plane. In one arrangement, the handle may be demountable such that it can be removed before sawing is carried out so that it does not impede access to a sawing guide. In one arrangement the handle may be formed integrally with the arm but may be demounted by breaking a frangible flange. The handle and the laser may be used by the surgeon to adjust the valgus varus position of the jig to meet the hip centre. In one arrangement the hip centre may be that as provided by the so-called Freeman 9cm rule. In another arrangement the valgus varus position may be determined by use of an electronic local guide unit comprising an accelerometer and gyroscope which locates the femoral head centre of the leg rotation. In a still further arrangement the varus-valgus position may be found by use of an intermedullary alignment rod inserted through the femoral jig and into the femoral canal.

In one arrangement the cutting guide may be a slot passing through the arm. In this arrangement, the handle may be mounted into the slot for use and then removed to expose the saw guide when sawing is required. The arm may also fit an extendible rod to assess flexion of the jig in the extended hip. In another arrangement, the cutting guide may be mounted on the handle by any suitable means.

Once the surgeon is happy with the position of the jig it is desirable that the jig is fixed to the femur to prevent movement from the selected positions in all freedoms, including medial/lateral position, during a trial stage. This can be achieved by any suitable means. In one arrangement, captive pins may be provided within the jig which can be impacted into position. Generally pins may be provided at the end of the arm and two on the face of the jig.

The jig may be made of any suitable material. Whilst it may be made of metal, in one arrangement it may be made of plastics material. Any suitable plastics material may be used. The jig may be injection moulded.

The surgeon will generally be able to assess the most likely size required from a simple x-ray or from assessing the patient's knee either pre-operatively or once the soft tissue has been cut. The operating theatre can then choose, or be provided with, a femoral jig of the correct size and optionally one size smaller or larger in case the surgeon wants to check whether a smaller or larger size would be suitable.

Once the jig is in position, the jig allows the surgeon to fit extra spacers if needed to test for joint space with the tibia in flexion and extension and in position between the two. The surgeon can then readily choose to change the size of the prosthesis or a component thereof as appropriate, adjust the positioning of the jig and hence the eventual prosthesis on the bone and if appropriate make decisions on treatment of the ligaments to correct any deformity.

The femoral jig will generally be put in position after resection of the tibia has occurred. Once placed against the femur while it is in relative flexion, the jig is rotated on the femoral bone until the anterior meets the femoral shaft. The position and size medial-lateral is confirmed and a view is taken on whether a smaller or larger jig would be a better fit for medial-lateral width and for alignment as viewed from laterally. Once a check on femoral valgus-varus has been carried out in extension as discussed above, flexion and extension space and function can be evaluated. A spacer representing the a tibial replacement guide can be used to assess the ideal tibia thickness.

According to a second aspect of the present invention there is provided a tibial replacement spacer comprising a set of leaves. In use the leaves can be stacked to judge tibia thickness. In one arrangement the leaves may be joined at the end remote from the end placed on the tibia. Each leaf may be of any suitable thickness. In one arrangement, each leaf will be about 2 mm thick. One end of each leaf may be sized to match, or be similar to, the end of the femur and they may be colour coded.

Fitting one leaf spacer in flexion provides the thinnest tibia. If the thinnest leaf spacer cannot be fitted more tibia will need to be removed or the thinnest prosthesis cannot be used. If the leaf can be fitted in flexion but not extension, soft tissue or more distal bone will need to be removed by an appropriate amount. Increasing the number of leaf spacers used enables the surgeon to test what actual tibia thickness should be used.

Once this has been done femoral peg drill holes are produced through the distal femur condyles. To facilitate this, the jig will generally include drill holes on the face of the component. The distal cut can then be made on the femur leaving sufficient depth of distal drill holes to fit the femoral component and second femoral cutting guide in a conventional manner.

The femoral jig can then be removed. The distal cut can be completed and a further distal cut can be made if it was so determined during balancing. In a preferred arrangement, particularly where it is made from plastics, the femoral jig can be discarded. A second femoral cutting guide in the form of a multi-cut block may then be located in the pre-formed peg holes to enable the surgeon to make the remaining cuts.

In a third aspect of the present invention there is provided a tibial jig for use in knee surgery, said jig comprising a component having tibial plate and tibial posts extending downwardly from said tibial plate and an anterior reference arm; said arm having a cutting guide located thereon.

The tibial jig will generally be provided as either for a left or right knee and generally or one size (by area). Thus it will allow access to the medial side for attachment and sawing through the cutting guide.

In general the tibial posts are shaped such that in use the jig references the condylar bottom. The posts can therefore be considered as providing feet. They may be of any suitable size but in one arrangement will have a diameter of about 8mm.

The reference arm may be of any suitable configuration but is generally shaped to hang down the tibia with a reference surface on the tibia tubercle and is suitable shaped with a step to help find the midline of the tibia s being through the medial third of the tibia tubercule.

The jig optionally includes a handle extending outwardly from the reference arm. The handle may facilitate the surgeon in adjusting the positioning of the jig. In one arrangement, a laser may be mounted on the handle or a mounting means for a separate laser may be provided. In one arrangement, the handle may be demountable such that it can be removed before sawing is carried out so that it does not impede access to a cutting guide. In one arrangement the handle may be formed integrally with the arm but may be demounted by a breaking a frangible flange. The handle and the laser may be used by the surgeon to adjust the position of the jig such that the laser points at the ankle centre just anterior to the joint. This allows setting of flexion at 5-7 degrees and straight valgus-varus leg alignment.

In one arrangement a rod may be used such that the orientation can be assessed in flexion. In this arrangement the jig may include means to allow a rod to be connected thereto such that it extends away from the knee joint in the direction of the foot. In one arrangement the rod may clip to the jig. In an alternative arrangement the jig may include an aperture through which the rod may pass to be inserted into a well in the end of the femur.

In one arrangement the cutting guide may be a slot passing through the arm. In this arrangement, the handle may be mounted into the slot for use and then removed to expose the cutting guide when sawing is required.

Once the surgeon is happy with the size and position of the jig it is desirable that it is fixed to the tibia to prevent movement from the selected position. This can be achieved by any suitable means. In one arrangement, captive pins may be provided within the jig which can be impacted into position. Generally pins may be provided at the end of the arm and two on the plate.

The jig may be made of any suitable material. Whilst it may be made of metal in one arrangement it may be made of plastics material. Any suitable plastics material may be used. The jig may be injection moulded. The jig may be disposable.

Holes in the plate provide drill guides and/or slots. There are preferably 3 drill guides.

The slot cut provides a single slot cut at nominal thickness from the highest point found by the feet.

According to a fourth aspect of the present invention there is provided a kit comprising one or more of a femoral jig according to the above first aspect of the present invention, a tibial replacement spacer according to the above second aspect of the present invention, a tibial jig according to the above third aspect of the present invention.

In one arrangement the kit may additionally comprise one or more of a multi-cut block, a re-cut block, a laser and a drill. Where the kit includes a tibial jig it may additionally include a rod. Even if all components are present the kit comprises far fewer components than were required in the prior art arrangements. The components may be packed in a single sterile pack. Since all components except the laser are size-specific, the laser may be packed separately. In one arrangement components of a particular size will be colour coded.

In one arrangement a kit for the femoral components will be provided and a separate kit of tibial components will be provided.

Even if all components of the femoral elements are present the kit only comprises 6 components. Similarly even if all components of the tibial elements are present, the kit will only comprise 4 components may be packed in a single sterile pack.

With this arrangement theatres can have the pre-op selected size available and other sides and sizes available outside theatre in single side and size packed in case they are required. Opening two or even three packs will not have a substantial cost implication. In one arrangement, a kit may be provided with small variation in sizes.

The present invention offers various advantages. The plan of the size and position of the components can be carried out by the surgeon during the operation. Since the surgeon is using his judgement, de-skilling is avoided. However, the decisions that the surgeon has to make are made easier by the function of the instruments of the present invention. Whilst pre-operative templating may be recommended it is not essential. The need for expensive and time-consuming scans is obviated. There is no requirement for the use of the invasive rods in the femur. The overall theatre time is reduced.

Further advantages include that only the correct side and size instruments and implants for each case need to be ready in the theatre sterile area. All instruments may be provided pre-sterile and may be disposable. A reduced number of instruments are required. The apparatus enables the flexion and extension gaps to be matched and fully balanced for ideal knee function. In addition, it is possible to fine tune the medial-lateral femur to a variable flexion of femur to provide ideal posterior referencing with ideal anterior referencing to optimize balance, joint line, and avoid anterior over stuffing.

The prepacked arrangement means that there is no risk of theatre/supplier mix-up. Further in a preferred arrangement there is no return of instruments and no separation of instruments from sets. No femur or tibia trials are required.

Of particular advantage to the patient is mid-stance laxity may be tested and ideal soft tissue balance can be tested and corrected. The patients therefore have more consistent outcomes whatever the implant type.

The present invention will now be described by way of example with reference to the following figures in which:
- Figure 1: is a perspective view of a prior art femoral guide;
- Figure 2: is a perspective view of a prior art tibial guide;
- Figure 3: is a perspective view of a femoral jig of the present invention;
- Figure 4: is a perspective view of the femoral jig of Figure 3 in position on a femur;
- Figure 5: is a side view of the arrangement of Figure 4;
- Figure 6: is a side view from the distal end of the arrangement of Figure 5;
- Figure 7: is a view from above of the arrangement of Figure 5;
- Figure 8: illustrates a first step of the operation;
- Figure 9a: illustrates a second step of the operation;
- Figure 9b: illustrates the jig position after completion of the second step;
- Figure 10: illustrates a third step of the operation;
- Figure 11: illustrates a fourth step of the operation;
- Figure 12: illustrates a fifth step of the operation;
- Figure 13a: illustrates a sixth step of the operation;
- Figure 13b: illustrates the configuration of the tibia after completion of the sixth step;
- Figure 14a: illustrates a seventh step of the operation;
- Figure 14b: illustrates the location of the jig after completion of the seventh step;
- Figure 15a: illustrates an eighth step of the operation;
- Figure 15b: illustrates the eighth step using an alternative location device;
- Figure 16: illustrates a ninth step of the operation;
- Figure 17: illustrates a tenth step of the operation;
- Figure 18: illustrates a eleventh step of the operation;
- Figure 19a: illustrates a twelfth step of the operation;
- Figure 19b: illustrates the resected bones;
- Figure 20: illustrates a thirteenth step of the operation and;
- Figure 21: illustrates the cuts made in the thirteenth step.

As illustrated in Figure 1, the femoral jig 1 comprises a component 2 that is shaped to represent the overall profile of a femoral prosthetic component of a specific side and size. That is to say its external profile substantially corresponds to the shape and size of the finished prosthesis. The component 2 has an anterior reference arm 3 which extends from the component and which includes a cutting guide 4. In the illustrated arrangement the cutting guide 4 is a slot extending through the arm 3.

The reference arm includes adjustment means 5 to allow the surgeon to visualize the fit of a prosthesis one size up anteriorly. In the illustrated arrangement the adjustment means is a screw. Captive pins may be provided in apertures 6 such that once the jig is in position the pins can be impacted to lock the jig in position. Apertures 7 provide drill guides.

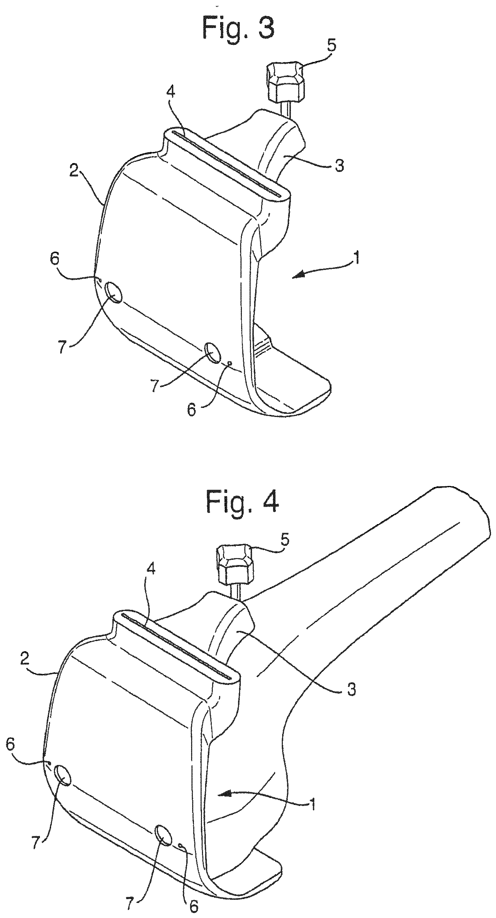

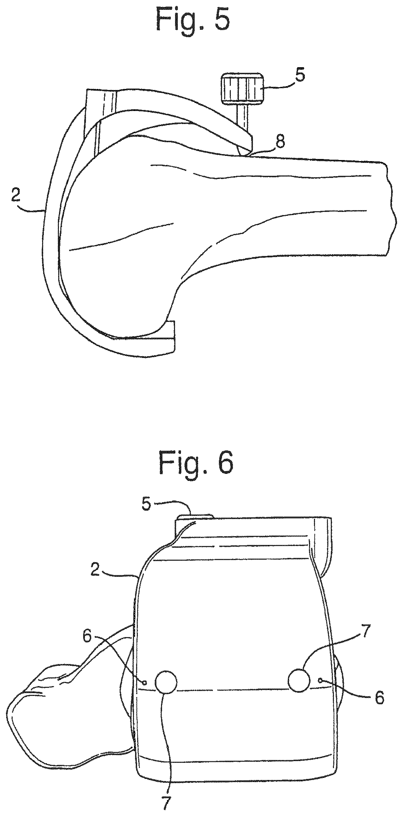

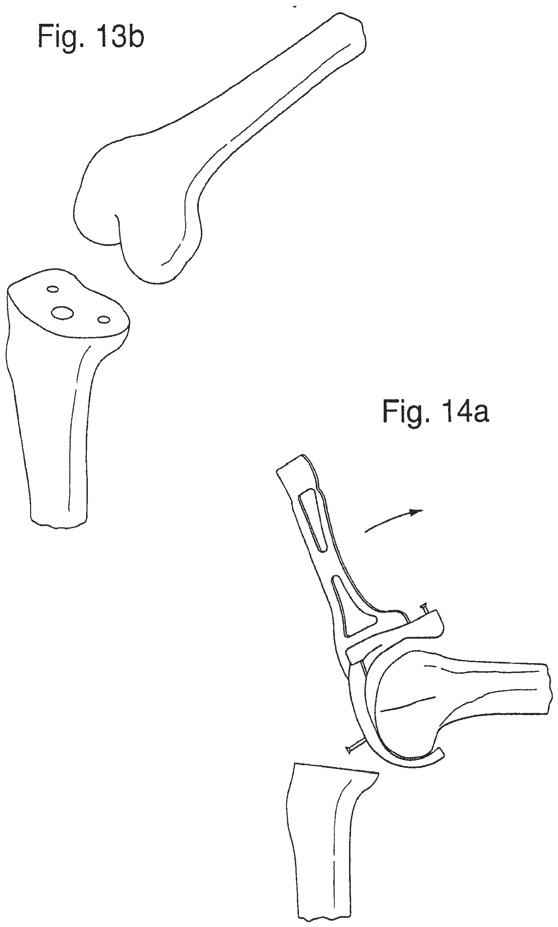

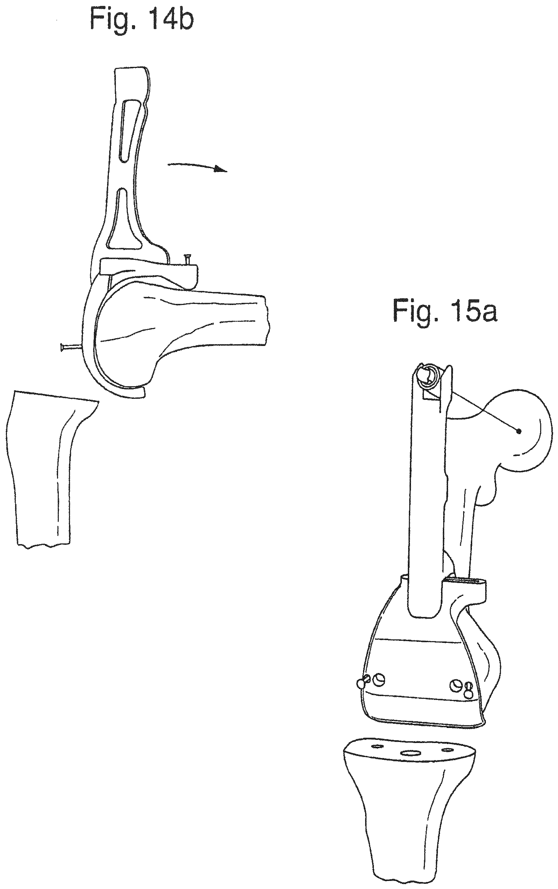

In use the surgeon offers up the jig to the exposed femur prior to any resection. This is illustrated in Figures 4, 5, 6 and 7. As illustrated in Figure 5, the tip 8 of the adjustment means 5 locates against the femur. Adjusting the adjustment means 5 will cause the jig to move around the end of the femur until it is in the required position. The effect of this on the alignment of the jig is further illustrated in Figures 14a and b. The pins 10 can then be driven into the bone to hold the jig in this desired position. Cuts can then be made through the cutting guide and holes can be drilled into the bone through the drill guides 7. Thus the jig ensures that the cuts and holes are in the optimum position.

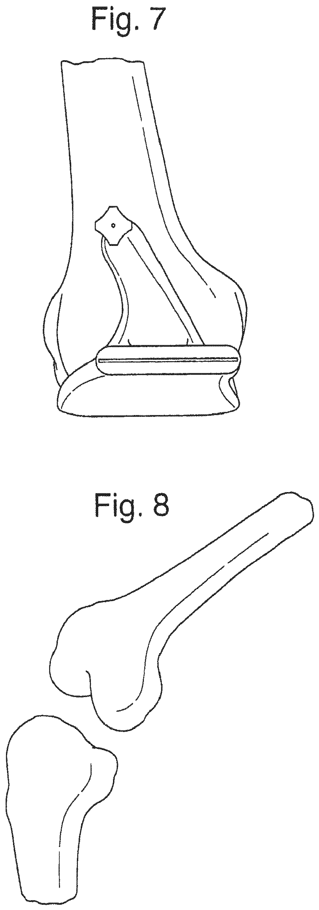

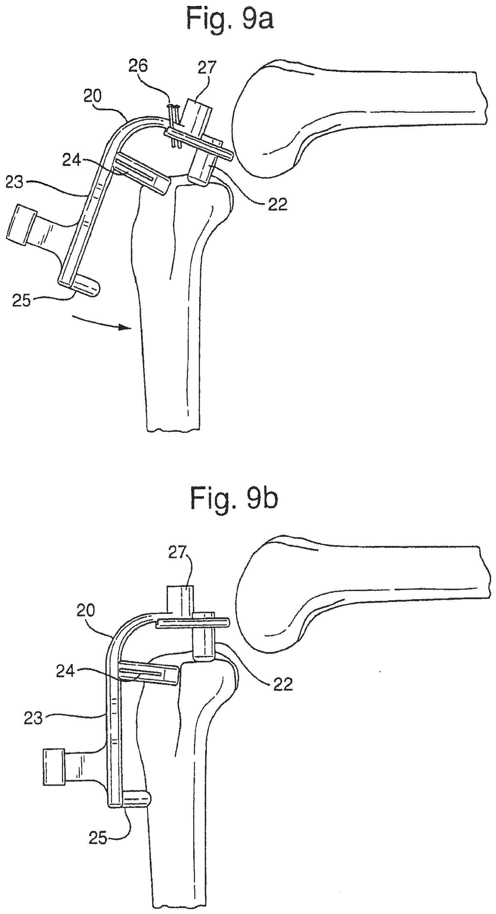

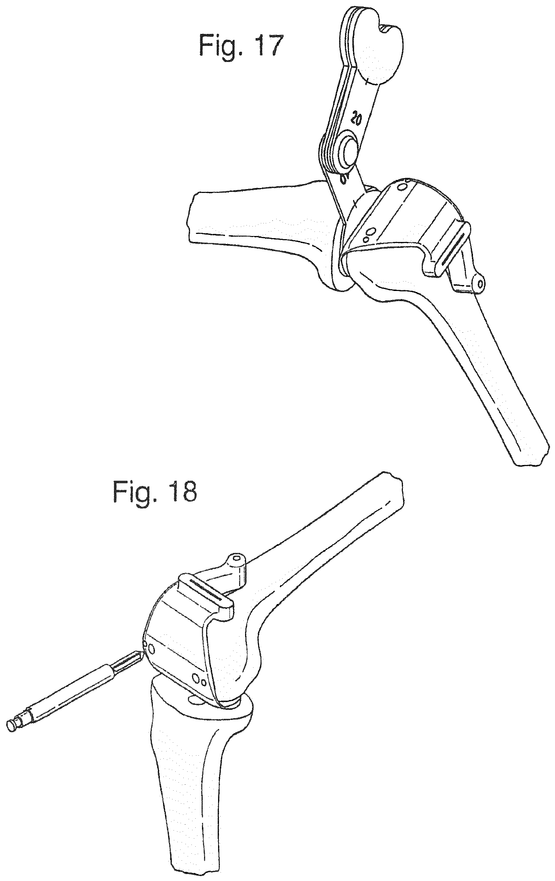

A full knee replacement utilising the apparatus of the present invention will now be described with reference to Figures 8 to 21. The first step is to expose the knee and check the templated measurements to produce a separated joint as illustrated in Figure 8.

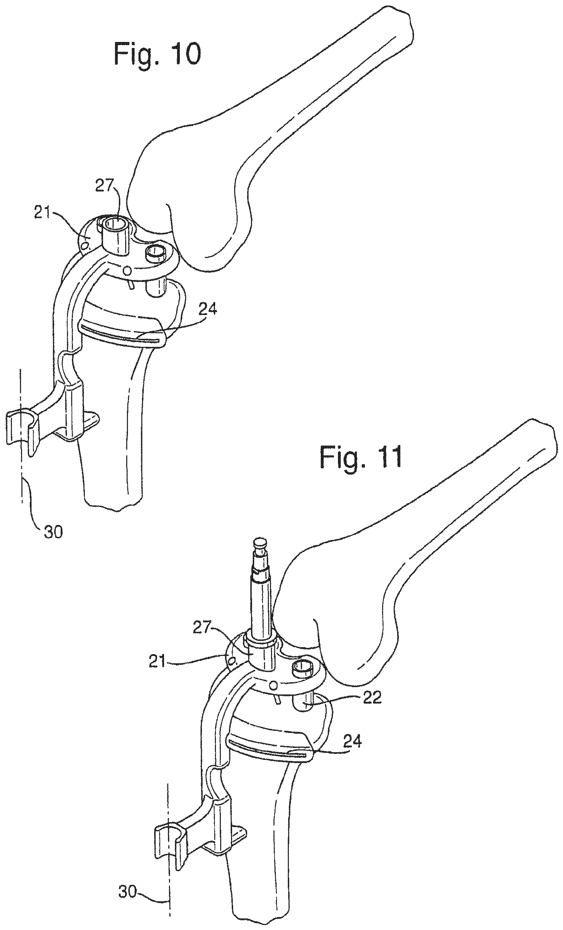

The tibial jig 20 comprises a tibial plate 21 (illustrated clearly in Figure 10) and tibial posts 22 extending downwardly from said tibial plate. The jig additionally includes an anterior reference arm 23 which has a cutting guide 24 located thereon. This tibial jig 20 is located on the head of the tibia such that the tibial posts are located on the tibial centres and a tail 25 extending from the arm interacts with tibia. Although a tail is used in the illustrated embodiment, the arm may have any appropriate configuration provided that it allows the jig to take up the optimal position on the tibia.

The alignment of the jig can then be checked. The surgeon may do this by eye or by other means. In one arrangement he may use a laser located on the tibia to provide a reference or a rod 30 may be clipped to the jig such that the relative alignment to, for example, the foot can be checked. Once the surgeon is happy with the orientation, pins 26 can be driven into the bone to hold the jig in position. The pins are shown in the driven in orientation in Figure 10.

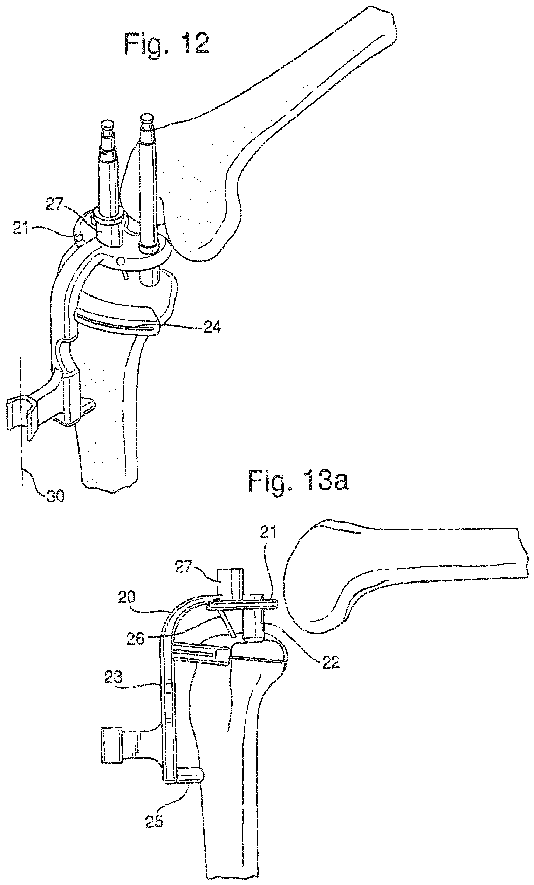

The jig will preferably include apertures 27 extending through the tibial posts. Drilling can occur through these apertures. The tibia can then be cut as illustrated in Figures 13a and b with the cutting guide enabling the appropriate cut to be made. The jig can then be removed and the head of the tibia will be removed with it to provide the sectioned bone illustrated in Figure 6.

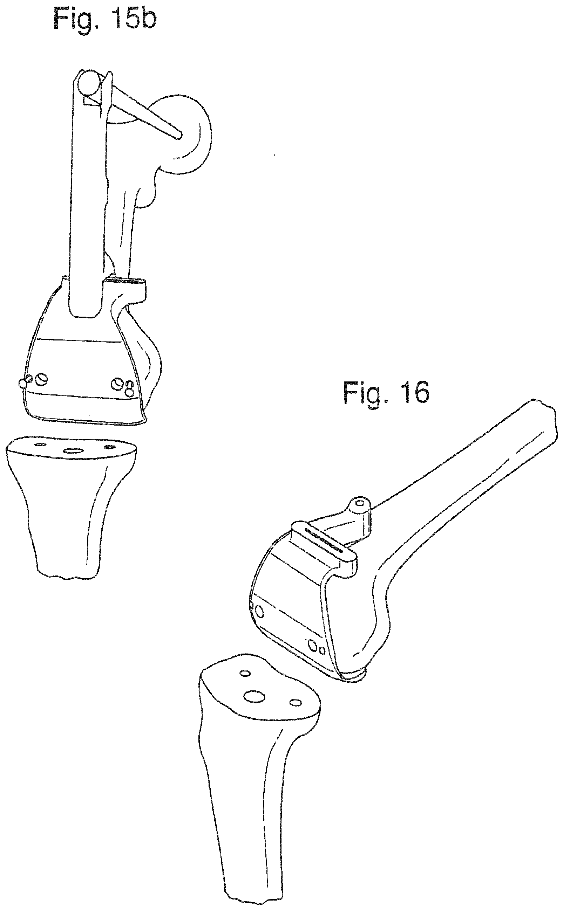

The surgeon will then turn his attention to the femur as illustrated in Figures 14a and b. The femoral guide, such as that illustrated in Figure 3 is placed on the femur and rotated to bring the anterior reference into contact with the lateral ridge of the femur. The jig illustrated in Figures 14a and b differs from that of Figure 3 in that it includes a handle which facilitates in the rotation of the jig. A laser 41 is located on the jig and when the jig is located in approximately the right position, the orientation can be checked using the beam from the laser to check against other elements on the body. In particular it can be used to check the varus/valgus alignment as illustrated in Figure 15a. In an alternative arrangement the jig may be replaced with a rod as illustrated in Figure 15b.

Once the jig is in the appropriate alignment, the handle may be removed and the jig pinned in place. In the arrangement illustrated in Figure 16, the jig is additionally pinned at the end of the arm.

The flexion and extension gaps can then be assessed utilising the tibial replacement spacer 50. This spacer comprises a plurality of leaves joined at one end. The leaves have a head 51 which is sized and shaped to sit on the cut tibia. Ligament release can then be conducted as required.

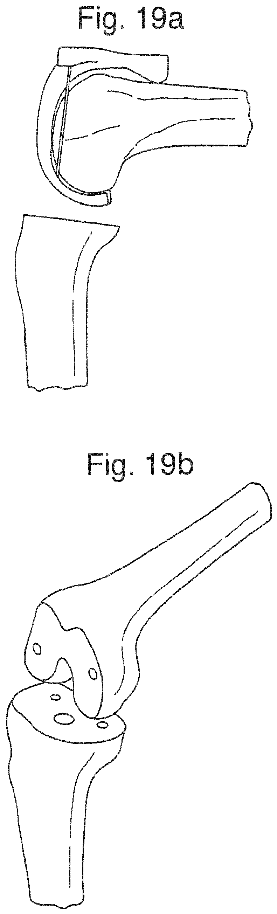

The femur can then be drilled with the apertures 7 providing appropriate guides. As illustrated in Figures 19a and 19b, the distal femur can then be cut.

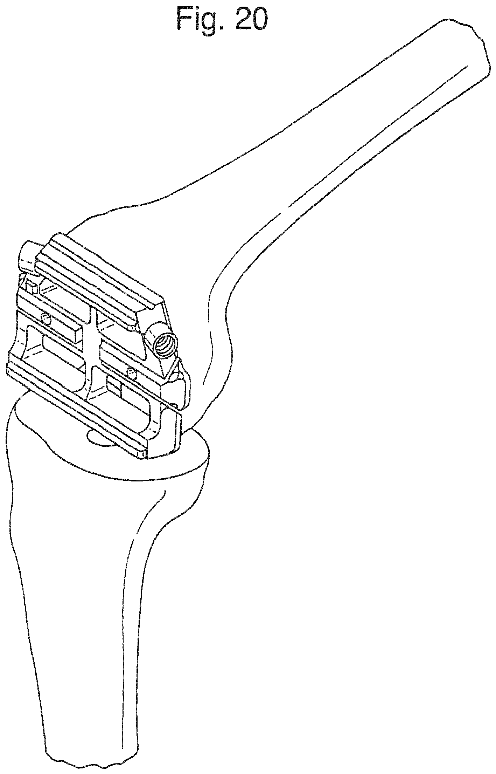

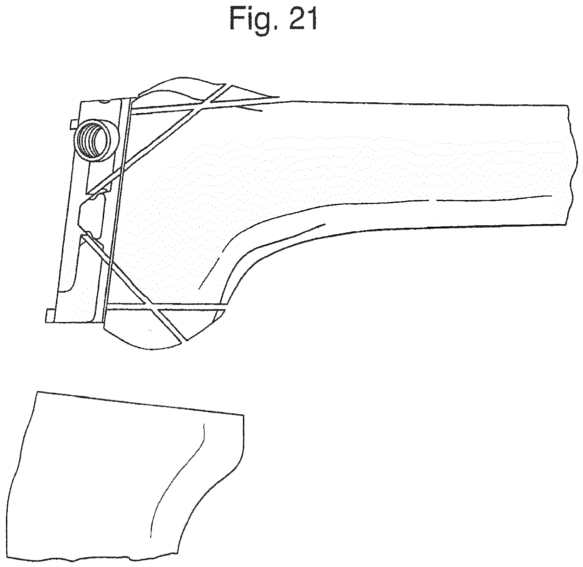

A multi-cut block such as that shown in Figure 20 can then be located on the resected femur. This can be placed in the correct orientation using the holes drilled into the femur. The block provides a guide for anterior and posterior cuts and for chamfers as illustrated in Figure 21.

## Claims

1. A tibial jig for use in knee surgery, said jig comprising a component having tibial plate and tibial posts extending downwardly from said tibial plate and an anterior reference arm; said arm having a cutting guide located thereon.

2. A tibial jig according to Claim 1, wherein the reference arm is shaped to hang down the tibia with a reference surface on the tibia tubercle and its medial side hooked onto with a step to find the midline of the tibia.

3. A tibial jig according to Claim 1 or 2, wherein the jig includes a handle extending outwardly from the reference arm.

4. A tibial jig according to any one of Claims 1 to 3, wherein a laser is mounted on the handle or a mounting means for a separate laser is provided.

5. A tibial replacement thickness spacer comprising a set of leaves.

6. A tibial replacement spacer wherein the leaves are joined at the end remote from the end placed on the tibia.

7. A femoral jig for use in knee surgery, said jig comprising a component shaped to represent the overall profile of a femoral prosthetic component of a specific side and size, and having extending therefrom an anterior reference arm; said arm having a cutting guide located thereon or means for attaching a cutting guide thereto.

8. A jig according to Claim 7, configured as being either for a left knee or for a right knee; and/or optionally wherein the thickness of the jig is selected to have both posterior and distal thicknesses correct for 3 degrees external rotation.

9. A jig according to any one of Claims 7 or 8, wherein the reference arm is shaped to sit at the ideal proximal lateral femoral component position on the femur ridge.

10. A jig according to Claim 9, wherein the reference arm includes adjustment means to allow the surgeon to visualize the fit of a prosthesis one size larger anteriorly; and optionally wherein the adjustment means is a screw.

11. A jig according to any one of Claims 7 to 10 wherein the jig includes an optionally demountable handle extending upwardly from the jig; and optionally wherein a laser is mounted on the handle or a mounting means for a laser is provided.

12. A jig according to Claim any one of Claims 7 to 14, where a hip centre guide system is located on the jig or the jig is provided with means or mounting a hip centre guide system.

13. A jig according to any one of Claims 7 to 12 wherein the cutting guide is a slot passing through the arm.

14. A jig according to any one of Claims 7 to 13, wherein the jig includes captive pins; and or optionally wherein the jig includes drill holes on its distal face.

15. A kit comprising one or more of a tibial jig according to any one of Claims 1 to 4, a tibial replacement spacer according to Claim 5 or 6, and a femoral jig according to any one of Claims 7 to 14; and optionally comprising one or more of a multicut block, a re-cut block, a laser and a drill.
